# EUROPEAN PATENT APPLICATION

(11) **EP 2 433 582 A1**
(43) Date of publication of application: **28.03.2012**
(21) Application number: 10777513.2
(22) Date of filing: 06.05.2010
(51) Int. Cl.: A61B 18/12

(54) **HIGH-FREQUENCY TREATMENT DEVICE**

(30) Priority: 21.05.2009 JP 2009123077
(71) Applicant: Sumitomo Bakelite Co., Ltd., Shinagawa-ku Tokyo 140-0002 (JP)
(72) Inventor: ISHII, Yasuhisa, Akita-shi Akita 011-8510 (JP); HARATA, Shinetsu, Akita-shi Akita 011-8510 (JP)
(74) Representative: Vossius, Corinna
(86) International application number: PCT/JP2010/003111
(87) International publication number: WO 2010/134273

(57) **Abstract**

A high-frequency treatment instrument (10) includes a flexible tube (50) for insertion into a body cavity, an operating wire inserted through the flexible tube (50), a treatment element (60) provided at a distal end of the operating wire to treat a diseased part by a high-frequency current, and a manipulating unit (20) attached to a proximal end of the flexible tube (50) to axially move the operating wire to manipulate the treatment element (60), and to rotate the operating wire torsionally about its axis thus rotating the treatment element (60). The manipulating unit (20) includes a main body (22) connected to a high-frequency cable (70), a rotational manipulator (24) attached to a distal side of the main body (22) to rotate the operating wire, and an axial manipulator (26) attached to a proximal side of the main body (22) to axially move thereby causing the operating wire to axially move.

## Description

### TECHNICAL FIELD

The present invention relates to a high-frequency treatment instrument.

### BACKGROUND ART

High-frequency treatment instruments for medical use are employed for treatment of a diseased part in a body by applying a high-frequency current, and generally used with an endoscope. The high-frequency treatment instrument includes an elongate operating wire with a treatment element attached to its distal end portion, and a manipulating unit for axially moving the operating wire or rotating the same about its axis, on a proximal end. In use of such a high-frequency treatment instrument, a high-frequency cable connected to a high-frequency power source is connected to a high-frequency terminal of the manipulating unit, and a high-frequency current is applied to the diseased part through the operating wire for treatment of the diseased part such as resection.

This type of instruments can be found, for example, in patent documents 1 and 2.
In a snare instrument according to the patent document 1, a plug for applying a high-frequency current is provided on an axial manipulator (spool member) that can axially slide with respect to a manipulating unit (handle assembly). The handle assembly of this snare instrument includes a main body and a knob coaxially coupled to each other, so that rotating the knob about the axis causes the operating wire (shaft) and the treatment element (snare loop) to rotate (see Fig. 11 of the patent document 1).

In a high-frequency treatment instrument according to the patent document 2, a plug for applying a high-frequency current is provided in a main body (base portion), and an manipulating unit for rotating and axially moving the treatment element is attached to a proximal side of the main body. The manipulating unit includes a ring-shaped manipulating element attached to the base portion with a fixed interval therebetween, and a slider movable back and forth with respect to the manipulating unit. Upon rotating the slider and the manipulating element with respect to the base portion, the operating wire and the treatment element are caused to rotate.

### RELATED DOCUMENTS

### PATENT DOCUMENTS

[Patent Document 1] JP-A No. 2003-506135
[Patent Document 2] JP-A No. 2007-325721

### DISCLOSURE OF THE INVENTION

The high-frequency treatment instruments according to the patent documents 1 and 2, however, still have a room for improvement in terms of operability in moving back and forth or rotating the operating wire.
Regarding the snare instrument according to the patent document 1, since the high-frequency cable is connected to the axial manipulator, the self weight of the high-frequency cable is imposed on the axial manipulator when the axial manipulator is moved back and forth. Accordingly, the axial manipulator becomes heavier, in other words a greater axial force is required for moving the axial manipulator, which makes it difficult to subtly control the snare loop.

Regarding the high-frequency treatment instrument according to the patent document 2, since the high-frequency current plug is provided in a main body (base portion), the slider can be manipulated free from the self weight of the high-frequency cable, and hence better operability is obtained in terms of back and forth movement of the operating wire. However, since the ring-shaped manipulating element for rotating the operating wire about its axis is mounted on the proximal side of the main body (base portion) in the form of an assembly coupled with the slider, it is difficult to subtly control the rotation angle of the operating wire.

The present invention has been accomplished in view of the foregoing situation, and provides a high-frequency treatment instrument that allows an operating wire to be subtly controlled in a back and forth and rotational movement.

In an aspect, the present invention provides a high-frequency treatment instrument including a flexible tube to be inserted in a body cavity, an electrically conductive operating wire inserted through the flexible tube so as to move back and forth, a treatment element provided at a distal end portion of the operating wire and configured to treat a diseased part by application of a high-frequency current, and a manipulating unit attached to a proximal end portion of the flexible tube and configured to move the operating wire back and forth to thereby manipulate the treatment element, and to rotate the operating wire torsionally about a longitudinal axis thereof to thereby rotate the treatment element, wherein the manipulating unit includes a main body being attached a high-frequency cable that supplies a high-frequency current to the treatment element, a rotational manipulator rotatably attached to a distal side of the main body so as to rotate the operating wire about the longitudinal axis thereof, and an axial manipulator attached to a proximal side of the main body so as to axially move to thereby cause the operating wire to move back and forth.

In a specific embodiment of the present invention, the main body may include a conductive pipe made of a metal buried therein, the conductive pipe being electrically connected to the high-frequency cable and through which the operating wire is inserted, and the operating wire may include a cylindrical conductor that slides on the conductive pipe maintaining electrical connection to the conductive pipe.

In another specific embodiment of the present invention, the high-frequency treatment instrument may include a lead pipe made of a metal attached to an outer periphery of the proximal end portion of the operating wire, and the conductor may be provided on an outer surface of the lead pipe.

In still another specific embodiment of the present invention, the high-frequency treatment instrument may further include a mitigator that applies a biasing force to the operating wire in a direction opposite to an axial movement thereof.

In still another specific embodiment of the present invention, the treatment element may include a plurality of scissor pieces caused to open and close with respect to each other by a movement of the axial manipulator to move the operating wire back and forth, and the mitigator may apply the biasing force to the operating wire when the scissor pieces are made to open by a forward or backward movement of the operating wire.

In still another specific embodiment of the present invention, the operating wire may be caused to move backward or forward by the biasing force of the mitigator, from a maximum open state realized by the axial movement of the operating wire where an aperture of the scissor pieces is largest, and the scissor pieces may present an intermediate open state where the aperture is smaller than that of the maximum open state.

In still another specific embodiment of the present invention, the mitigator may be resiliently interposed between the main body and the axial manipulator.

In still another specific embodiment of the present invention, the mitigator may include a holder engaged with the operating wire with a predetermined engaging force to thereby restrict a back-and-forth movement of the axial manipulator, and a biasing member resiliently interposed between the holder and the axial manipulator, and the holder may become disengaged from the operating wire when a load greater than the predetermined engaging force is applied to the axial manipulator in the axial direction, to thereby allow the back-and-forth movement of the axial manipulator.

In still another specific embodiment of the present invention, the rotational manipulator may include a plurality of stepped portions formed on a proximal end portion thereof, the main body may include a multiple-stage fitting base formed on a distal end portion thereof, the fitting base being attached the rotational manipulator, and the plurality of stepped portions may make plane-to-plane contacts with the fitting base.

In still another specific embodiment of the present invention, the rotational manipulator may include a projection formed on the proximal end portion thereof, the projection being inserted through the fitting base, and the projection may include a stopper formed therearound so as to prevent the projection from coming off from the fitting base.

In still another specific embodiment of the present invention, the stopper may bias the rotational manipulator toward the proximal side of the main body.

In still another specific embodiment of the present invention, the axial manipulator may have an axially symmetrical shape.

In still another specific embodiment of the present invention, the high-frequency treatment instrument may further include an annular auxiliary ring pivotally attached to the main body and having an opening oriented in a direction intersecting with the axial direction, and the axial manipulator may be movable in the axial direction being restricted from rotating about the axis with respect to the main body.

In still another specific embodiment of the present invention, the rotational manipulator may be circumferentially exposed on an outer surface of the main body.

Here, the constituents of the present invention such as the manipulating unit and the high-frequency inlet do not always have to be individually independent, but may be arranged such that a plurality of constituents constitutes a unified member, that a plurality of members constitutes a constituent, that a constituent is a part of another constituent, or that a part of a constituent also serves as a part of another constituent.

In the high-frequency treatment instrument according to the present invention, the high-frequency cable is attached to the main body, and the rotational manipulator for rotating the operating wire about its axis and the axial manipulator for moving the operating wire back and force are provided independently from the main body. Accordingly, the operating wire can be moved back and forth or rotated free from the self weight of the high-frequency cable, which allows more subtle control of the operating wire compared with the snare instrument according to the patent document 1.
In addition, since the high-frequency treatment instrument according to the present invention is configured to apply a rotational torque to the operating wire on the distal side of the main body, the distance between the treatment element attached to the distal end of the operating wire and the position where the rotational torque is applied is shorter than in the high-frequency treatment instrument according to the patent document 2, in which a rotational torque is applied to the operating wire on the proximal side of the main body. Such a configuration allows the treatment element to more quickly respond to the rotational manipulation of the operating wire. Thus, the present invention allows the operating wire to be subtly controlled in a back and forth and rotational movement.

### BRIEF DESCRIPTION OF THE DRAWINGS

The above and other objects, features and advantages will become more apparent through preferred embodiments described hereunder and the accompanying drawings specified below.

Fig. 1 is a perspective view showing a high-frequency treatment instrument according to a first embodiment of the present invention;
Fig. 2 is a front view of the high-frequency treatment instrument according to the first embodiment;
Fig. 3(a) is a front view showing a treatment element in an open state, and Fig. 3 (b) is front view showing a treatment element in a closed state;
Fig. 4(a) is a vertical cross-sectional view of a manipulating unit, and Fig. 4(b) is a transverse cross-sectional view thereof;
Fig. 5 is an enlarged view of a portion of Fig. 4(a);
Fig. 6 is a cross-sectional view of a fitting base formed on a main body;
Figs. 7(a) to 7(c) are cross-sectional views of a manipulating unit of a high-frequency treatment instrument according to a second embodiment of the present invention, Fig. 7(a) showing a state where a slider is at a retreated position, Fig. 7(b) showing a state where the slider is at the forwardmost position, and Fig. 7(c) showing a state where an operating wire is slacked;
Fig. 8(a) is a side view of a distal side of a holder constituting a mitigator, and Fig. 8(b) is a front view thereof;
Fig. 9(a) is a vertical cross-sectional views showing a state before fitting the mitigator, and Fig. 9(b) is a vertical cross-sectional views showing a state after fitting the mitigator; and
Figs. 10(a) to 10(c) are cross-sectional partial views of a manipulating unit of a high-frequency treatment instrument according to a variation of the second embodiment, Fig. 10(a) showing a state where the slider is restricted frommoving forward, Fig. 10 (b) showing a state where the slider is moving forward, and Fig. 10(c) showing a state where the operating wire is slacked.

### DESCRIPTION OF EMBODIMENTS

Hereafter, embodiments of the present invention will be described referring to the drawings. In all the drawings, the same constituents will be given the same numeral, and the description thereof will not be repeated.

### [First embodiment]

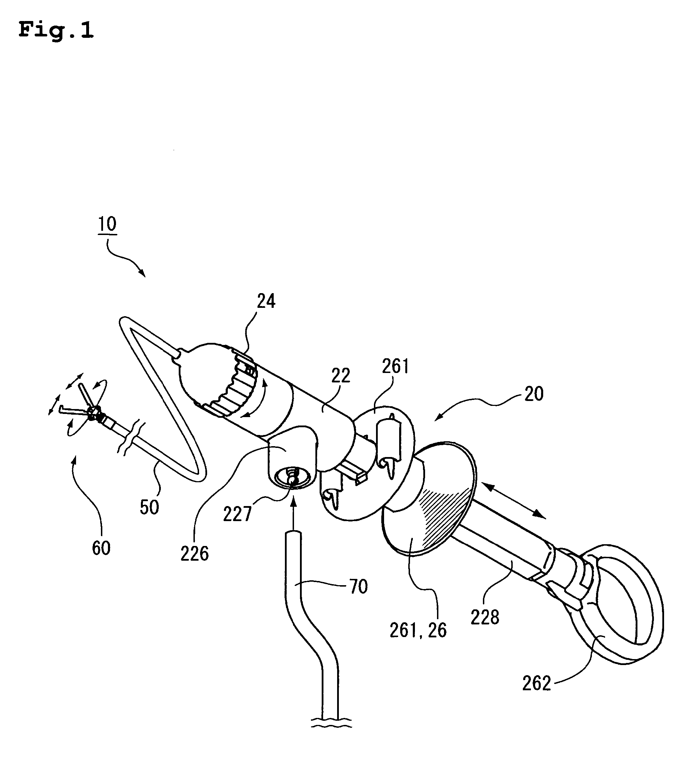

Fig. 1 is a perspective view showing a high-frequency treatment 10 instrument according to a first embodiment of the present invention the present invention. In Fig. 1, a portion of an elongate flexible tube 50 is cut away.

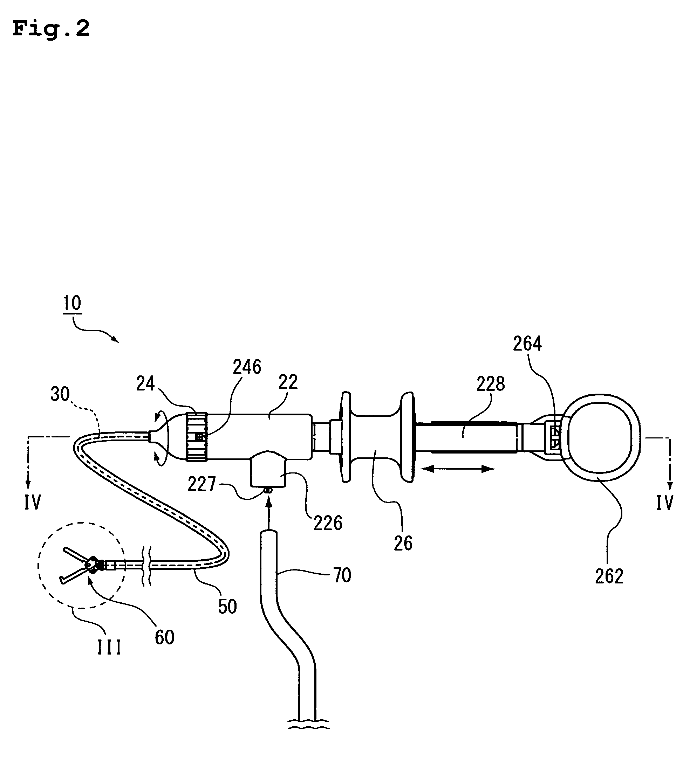

Fig. 2 is a front view of the high-frequency treatment instrument 10 according to this embodiment. In the subsequent description, a lateral direction with respect to a longitudinal direction of the high-frequency treatment instrument 10 will be referred to as a front direction.

To start with, an outline of the high-frequency treatment instrument 10 according to this embodiment will be described.
The high-frequency treatment instrument 10 according to this embodiment includes a flexible tube 50 to be inserted in a body cavity, an electrically conductive operating wire 30 inserted through the flexible tube 50 so as to move back and forth, a treatment element 60 provided at a distal end portion of the operating wire 30 and configured to treat a diseased part by application of a high-frequency current, and a manipulating unit 20 attached to a proximal end portion of the flexible tube 50.
The manipulating unit 20 is a mechanism for moving the operating wire 30 back and forth thereby moving the treatment element 60 and for rotating the operating wire 30 torsionally about its axis thereby rotating the treatment element 60, and includes a main body 22, a rotational manipulator 24, and an axial manipulator 26.
A high-frequency cable 70 through which a high-frequency current is supplied to the treatment element 60 is connected to the main body 22. The rotational manipulator 24 is rotatably attached to a distal side of the main body 22 so as to rotate the operating wire 30 about the axis thereof. The axial manipulator 26 is attached to a proximal side of the main body 22 so as to axially move, to thereby cause the operating wire 30 to move back and forth.

Further details of the high-frequency treatment instrument 10 according to this embodiment will now be described.
The treatment element 60 employed in the high-frequency treatment instrument 10 moves interlocked with the back and forth movement of the operating wire 30, and rotates interlocked with the rotation of the operating wire 30 about its axis. The treatment element 60 serves to treat a diseased part by application of a high-frequency current.

Although the configuration of the treatment element 60 is not specifically limited, this embodiment adopts a scissors type treatment element for incising a diseased part utilizing a linkage mechanism. Alternatively, a conventionally known device such as a knife type with a bent point or a wire snare loop may be employed as the treatment element 60. The scissors type treatment element 60 includes, as will be subsequently described, a linkage mechanism driven by a back and forth movement of the operating wire 30, and incises a diseased part while applying a high-frequency wave. In the case of the knife type treatment element, the bent point is hooked on a diseased part, and the treatment element is pulled toward the proximal side while applying a high-frequency wave, to incise the diseased part. In the case of the snare treatment element, the diameter of the snare is increased and decreased by a back and forth movement of the operating wire 30, so as to bind a diseased part.

The operating wire 30 according to this embodiment is a wire rod made of a metal. The operating wire 30 is inserted through the flexible tube 50 constituted of a flexible material.
The material of the flexible tube 50 is not specifically limited, and either a conductive or non-conductive material may be employed. In the case of employing a conductive material, it is preferable to provide a thin-film coating of an insulative material on the conductive inner surface of the flexible tube 50.
Examples of the non-conductive material suitable for the flexible tube 50 include resin materials such as a fluorine resin, a polyimide resin, a polyethylene resin, a polypropylene resin, a polyurethane resin, a polyamide resin, and a polycarbonate resin. In particular, a fluorine resin is preferable because of excellent slidability with respect to a forceps channel through which the flexible tube 50 is inserted.

The treatment element 60 sticks out from the distal end opening of the flexible tube 50. The treatment element 60, the operating wire 30 and the flexible tube 50 are inserted through a lumen of an endoscope (not shown).

The manipulating unit 20 is a mechanism for pulling and pushing the operating wire 30, or torsionally rotating the same about a longitudinal axis thereof. The manipulating unit 20 according to this embodiment essentially includes a main body 22, a rotational manipulator 24, and an axial manipulator 26.

The main body 22 has a hollow cylindrical shape, and accommodates therein the proximal end portion of the operating wire 30. The axial direction of the main body 22 and the extending direction of the operating wire 30 coincide with each other. Accordingly, the term "axis" or "axial" herein refers to the extending direction of the operating wire 30, unless otherwise defined specifically.

The rotational manipulator 24 is attached to the distal side of the main body 22. The rotational manipulator 24 is engaged with the operating wire 30, such that upon rotating the rotational manipulator 24 about the axis the treatment element 60 is caused to rotate about the axis, as indicated by curved arrows in Figs. 1 and 2.

The rotational manipulator 24 is located on the distal end portion of the manipulating unit 20, more particularly on the distal side of the main body 22. The term "distal end portion" of the manipulating unit 20 herein refers to a longitudinal region having a predetermined extension.
In the high-frequency treatment instrument 10 according to this embodiment, the rotational manipulator 24 is located at the tip end of the manipulating unit 20.

The rotational manipulator 24 is attached over the distal side face of the main body 22 in a cap shape. The rotational manipulator 24 is circumferentially exposed on the outer periphery of the main body 22.
The rotational manipulator 24 includes a corrugated knurling formed along the outer circumferential surface thereof, the outer circumferential surface about the axis of the manipulating unit 20.

The high-frequency treatment instrument 10 according to this embodiment can be accessed by an operator from any direction about the axis with respect to the rotational manipulator 24. Since the rotational manipulator 24 is circumferentially exposed on the outer periphery of the main body 22, the rotational stroke of the rotational manipulator 24 is unlimited. In the case where only a part of the outer periphery of the knob is exposed, as the handle assembly of the snare instrument according to the patent document 1, it is difficult to continuously rotate the treatment element 60 over a predetermined rotational angle (for example, 180 degrees). More specifically, to rotate the treatment element by an angle exceeding 180 degrees when operating the snare instrument according to the patent document 1, the operator has to once remove his/her finger from the knob upon having rotated the knob to the end of the rotational stroke, and then move the finger to the start point of the rotational stroke in order to resume the rotating operation. Thus it is impossible to continuously manipulate the knob, which restricts the operator from delicately rotating the treatment element.
In contrast, with the high-frequency treatment instrument 10 according to this embodiment, since the rotational stroke of the rotational manipulator 24 is unlimited, the treatment element 60 can be made to continuously rotate irrespective of the desired rotational angle of the treatment element 60.

As indicated by straight arrows in Figs. 1 and 2, upon axially sliding the axial manipulator 26 with respect to a shaft 228 of the main body 22 the treatment element 60 is caused to open and close.

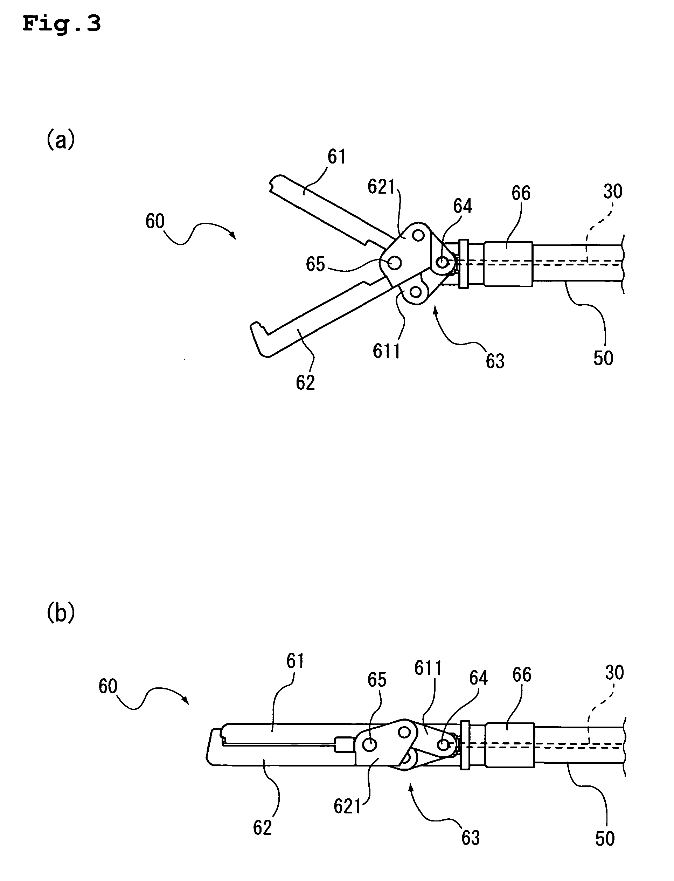

Figs. 3(a) and 3 (b) are enlarged views of a portion III circled by broken lines in Fig. 2. Fig. 3(a) is a front view showing the treatment element 60 in an open state, and Fig. 3 (b) is a front view showing the treatment element 60 in a closed state.

As shown in Figs. 3(a) and 3(b), the treatment element 60 according to this embodiment includes a first scissor piece 61 and a second scissor piece 62 configured to open and close with respect to each other about a first pin 65.
The operating wire 30 slidably inserted through the flexible tube 50 is connected to the base portion 66 of the treatment element 60. When the operating wire 30 is pushed toward the distal side (to the left in Figs. 3(a) and 3(b)), a second pin 64 is biased toward the first pin 65, so that a linkage mechanism 63 causes the treatment element 60 to open.
The linkage mechanism 63 is a four-bar linkage mechanism including a first link plate 611 formed in the base portion of the first scissor piece 61 and a second link plate 621 formed in the base portion of the second scissor piece 62.
Upon pushing the operating wire 30 toward the distal side the first scissor piece 61 and the second scissor piece 62 pivot with respect to each other thus to open as shown in Fig. 3(a).

Fig. 3(a) illustrates a state where the first scissor piece 61 and the second scissor piece 62 are opened to a maximum angle. When the operating wire 30 is continuously moved forward, an upper limit of the opening angle of the first scissor piece 61 and the second scissor piece 62 is delimited by the maximum open state.

Upon pulling the operating wire 30 to the proximal side (to the right in Figs. 3(a) and 3(b)), the respective tip portions of the first scissor piece 61 and the second scissor piece 62 are closed by the action of the linkage mechanism 63, as shown in Fig. 3(b).

As shown in Figs. 1 and 2, the main body 22 includes a high-frequency inlet 226 electrically connected to the high-frequency cable 70. The high-frequency inlet 226 includes a power source terminal 227 to which the high-frequency cable 70 is to be attached. The power source terminal 227 is disposed so as to project in a direction orthogonal to the axis of the high-frequency treatment instrument 10.
Accordingly, the high-frequency cable 70 is connected to the main body 22 in a direction intersecting with the axial line.

In the high-frequency treatment instrument 10 according to this embodiment, the high-frequency inlet 226 is fixed to the main body 22. Upon connecting the high-frequency cable 70 (see Fig. 2) to the high-frequency inlet 226, the high-frequency cable 70 hangs down from the high-frequency inlet 226 because of its self weight. Accordingly, the rotational manipulator 24 and the operator can be prevented from getting tangled with the high-frequency cable 70, when the rotational manipulator 24 is operated so as to rotate the treatment element 60.

The axial manipulator 26 according to this embodiment has an axially symmetrical shape. The axial manipulator 26 is rotationally symmetrical about the axial line, and more specifically of a cylindrical shape with a recess circumferentially formed about the axial line that allows the operator to hold the axial manipulator 26 with the forefinger and middle finger. The axial manipulator 26 also includes a pair of flange portions 261 respectively formed on the proximal and distal sides thereof so as to radially protrude outwardly.

The high-frequency treatment instrument 10 according to this embodiment also includes an annular auxiliary ring 262 that can be rotated with respect to the main body 22 about the axis. The auxiliary ring 262 includes an opening extending in a direction intersecting with the axial line. The axial manipulator 26 is slidable back and forth but fixed to the main body 22 in terms of rotating motion.

The auxiliary ring 262 serves to assist the movement of the axial manipulator 26, as a fulcrum for sliding the axial manipulator 26 back and forth.
The auxiliary ring 262 is pivotally connected to the shaft 228 via a pivotal base 264. The operator can, for example, insert the thumb through the auxiliary ring 262 and use the forefinger and middle finger to axially slide the axial manipulator 26, to thereby move the operating wire 30 back and forth.

In the high-frequency treatment instrument 10 according to this embodiment, the auxiliary ring 262 is pivotable with respect to the main body 22, and the axial manipulator 26 has an axially symmetrical shape. Such a configuration allows the operator to hold the high-frequency treatment instrument 10 from any desired direction.
Regarding the high-frequency treatment instrument according to the patent document 2, since the holding orientation of the manipulating unit is specifically determined, it may be difficult to perform a rotational or back-and-forth operation depending on the posture of the operator. In addition, in the case of the high-frequency treatment instrument according to the patent document 2, since the treatment element is made to rotate upon rotating the manipulating unit about its axis, the treatment element may accidentally rotate during the operation of the manipulating unit.
In contrast, with the high-frequency treatment instrument 10 according to this embodiment, the treatment element 60 can be kept from rotating while the manipulating unit 20 is being handled, unless the rotational manipulator 24 is rotated. Further, since the axial manipulator 26 is accessible from a desired direction, the treatment element 60 can be made to rotate or move back and forth irrespective of the posture of the operator.

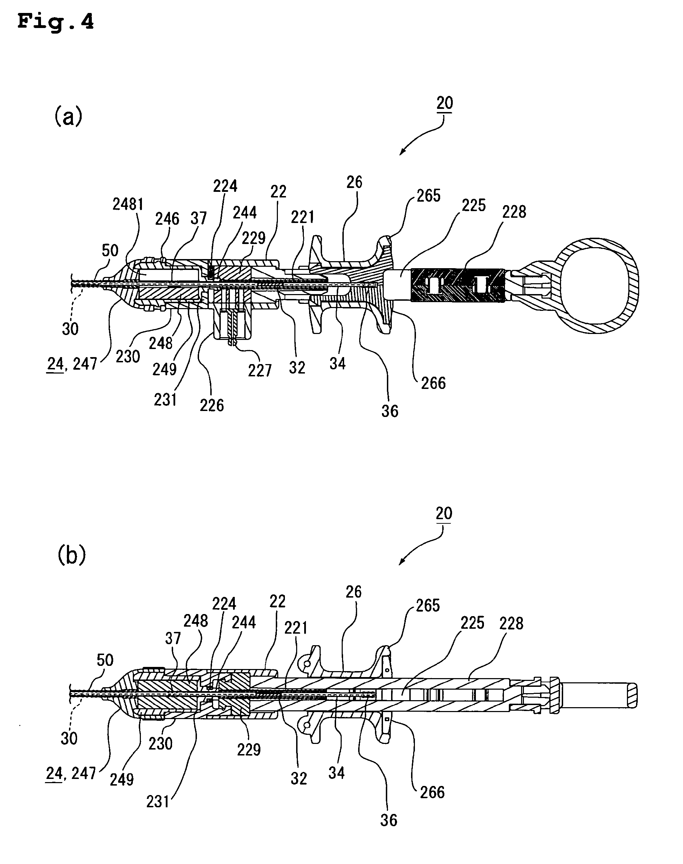

Fig. 4(a) is a vertical cross-sectional view of the manipulating unit 20 according to this embodiment, and Fig. 4 (b) is a transverse cross-sectional view thereof. Fig. 4(b) may also be taken as a vertical cross-sectional view of the manipulating unit 20 placed with the lateral side oriented upward. Here, the "vertical cross-section" of the manipulating unit 20 refers to a cross-section taken along a plane containing the axial line of the manipulating unit 20. In addition, Fig. 4 (b) is a cross-sectional view taken along a line IV-IV in Fig. 2.

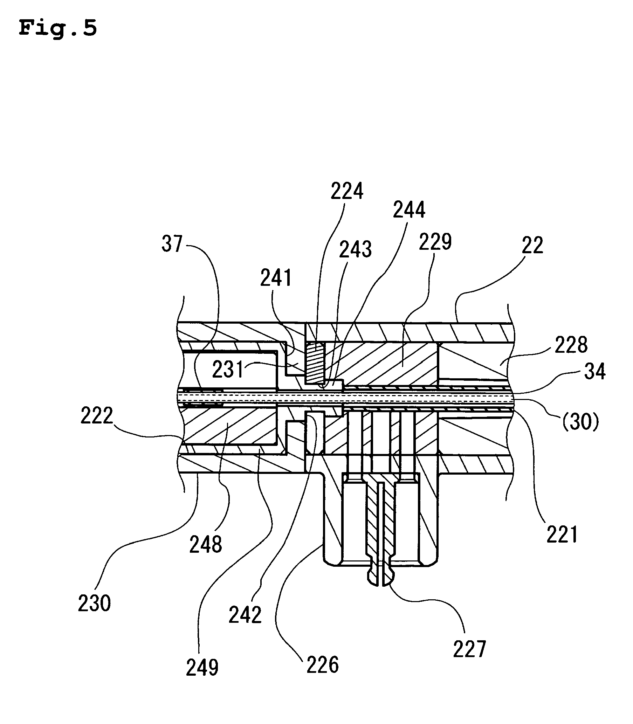

Fig. 5 is an enlarged view of a portion around the high-frequency inlet 226 in Fig. 4(a).

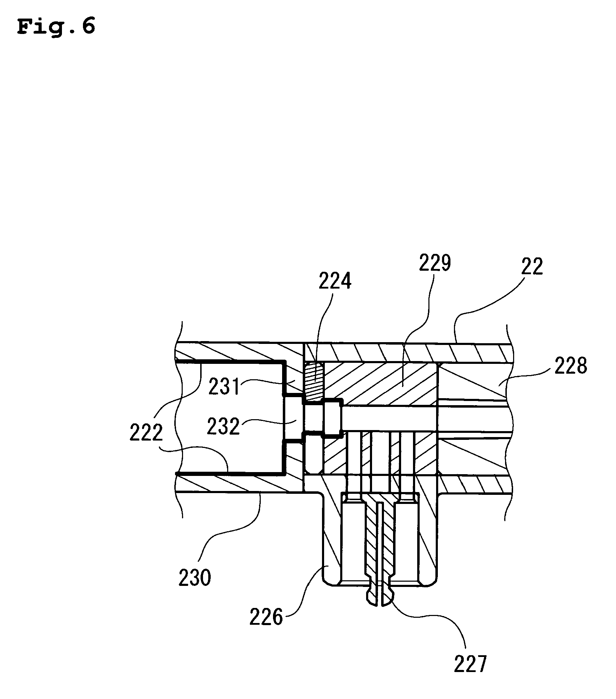

Fig. 6 is a cross-sectional view of a fitting base 222 formed on the main body 22.

The manipulating unit 20 includes a hollow portion at least on the distal side thereof, and the proximal end portion of the operating wire 30 is inserted along the axial center of the hollow portion.

Inside the manipulating unit 20, a lead pipe 34 made of a metal is attached to the outer circumference of the proximal end portion of the operating wire 30. The operating wire 30 and the lead pipe 34 are electrically and mechanically connected to each other. Thus, the operating wire 30 and the lead pipe 34 rotate about the axis interlocked with each other.
The lead pipe 34 is formed of a conductive metal. The thickness and outer diameter of the lead pipe 34 are not specifically limited. The lead pipe 34 attached to the outer circumference of the operating wire 30 serves to increase the torsional rigidity of the operating wire 30.

The rotational manipulator 24 according to this embodiment is composed of a combination of a cap 247, an engaging portion 248, and a base fitting portion 249, all of which are made of an insulative material.

The cap 247 is the portion to be manipulated by the operator, attached to the distal side of the main body 22.
The cap 247 has a generally hemispherical shape, and includes an orifice formed at a position corresponding to the axial center, through which the operating wire 30, the lead pipe 34 and the flexible tube 50 are inserted. The proximal end portion of the flexible tube 50 is fixed to the cap 247.

The base fitting portion 249 is a cylindrical member, having its proximal end attached to the main body 22 and its distal end coupled with the cap 247. In other words, the base fitting portion 249 serves as a fixing member that fixes the rotational manipulator 24 to the main body 22.
The base fitting portion 249 includes an opening of an increased diameter formed on the distal side, and a stepped cylindrical projection 244 of a reduced diameter formed on the proximal side.

The cap 247 includes a plurality of nail portions 246 formed at different positions on the outer periphery thereof. The nail portions 246 are engaged with the base fitting portion 249. Accordingly, the cap 247 and the base fitting portion 249 are restricted from moving relatively to each other, both in rotational and sliding motions.

The engaging portion 248 is accommodated inside the opening on the distal side of the base fitting portion 249 so as to rotate about the axis together with the cap 247 and the base fitting portion 249.
The engaging portion 248 accommodated inside the opening of the base fitting portion 249 is restricted from rotating about the axis with respect to the base fitting portion 249.

The engaging portion 248 includes a slit 2481 formed so as to axially extend, and hence has a channel shape having a U-shaped cross-section. The engaging portion 248 is attached to the lead pipe 34 from a radial direction.
The slit 2481 is formed in an upper region of the axial center of the engaging portion 248, according to the orientation of Fig. 4(a). In other words, the opening of the U-shaped engaging portion 248 is directed upward in Fig. 4(a).
The engaging portion 248 is engaged with the lead pipe 34 so as to apply a rotational torque thereto, and to cause the lead pipe 34 to axially slide.

In this embodiment, the lead pipe 34 includes an operating wire engaging portion 37 of a rectangular column shape formed so as to swell from the outer circumferential surface thereof, at a position inside the engaging portion 248.
The width of the slit 2481 of the engaging portion 248 is wider than the distance between opposite faces of the rectangular cross-section of the operating wire engaging portion 37, i.e., the width of the operating wire engaging portion 37, but smaller than the diagonal distance of the rectangular cross-section of the operating wire engaging portion 37.
Therefore, when the engaging portion 248 is rotated about the axis with the operating wire engaging portion 37 accommodated inside the slit 2481, the lead pipe 34 is caused to rotate about the axis.

A cylindrical connection cap 230 having an opening on the distal side is provided on the distal side of the main body 22. The connection cap 230 includes an inner flange 231 formed at the base portion thereof.
Through an opening 232 (see Fig. 6) defined by the inner flange 231, the projection 244 of the base fitting portion 249 is inserted.

The high-frequency inlet 226 and the power source terminal 227 are provided on the main body 22 so as to radially protrude therefrom. A power supply terminal 229 electrically connected to the power source terminal 227 is accommodated inside the main body 22. The power supply terminal 229 and the power source terminal 227 are electrically and mechanically connected to each other, by means of a metal screw (not shown).

The main body 22 contains thereinside a conductive pipe 221 made of a metal. The conductive pipe 221 is electrically connected to the high-frequency cable 70 (see Fig. 2), and the operating wire 30 is inserted through the conductive pipe 221.
A cylindrical conductor 32 that slides with respect to the conductive pipe 221 in electrical connection therewith is provided on the operating wire 30.

The conductor 32 is provided on the outer surface of the lead pipe 34, so as to slidably contact the inner surface of the conductive pipe 221.
A high-frequency current supplied to the power source terminal 227 through the high-frequency cable 70 is applied to the treatment element 60 through the conductive pipe 221, the conductor 32, the lead pipe 34, and the operating wire 30 (see Fig. 2).

In the high-frequency treatment instrument 10 according to this embodiment, the conductor 32 and the conductive pipe 221, both made of a conductive metal material, are slidably disposed and electrically connected to each other. Thus, in the high-frequency treatment instrument 10 according to this embodiment, the power source terminal 227 and the operating wire 30 are electrically connected to each other, substantially free from frictional connection. Accordingly, excellent conductivity can be achieved between the power source terminal 227 and the operating wire 30, without the need to apply an excessive axial force to push or pull the operating wire 30 with the axial manipulator 26.

As shown in Figs. 4(a) and 4(b), an operating wire fixing base 36 is provided at the proximal end portion of the lead pipe 34, in an expanded shape. The operating wire fixing base 36 is engaged with the axial manipulator 26 so as to rotate about the axis. When axial manipulator 26 is made to slide with respect to the shaft 228, the lead pipe 34 and hence the operating wire 30 are caused to move back and forth.

The shaft 228 includes a slit 225 formed so as to axially extend.
The axial manipulator 26 according to this embodiment is composed of a combination of a slider fixer 266 fitted in the slit 225 so as to axially slide with respect to the shaft 228, and a slider 265 attached to the outer periphery of the slider fixer 266. The operating wire fixing base 36 is buried in the slider fixer 266.

As shown in Fig. 5, a plurality of stepped portions 241, 242 is formed on the base portion of the rotational manipulator 24 (base fitting portion 249).
On the distal side of the main body 22, on the other hand, the inner flange 231 of the connection cap 230, a stopper 224, and the power supply terminal 229 constitute a multiple-stage fitting base 222 including a plurality of stepped portions, to which the rotational manipulator 24 is fitted, as indicated by bold lines in Fig. 6.
Thus, upon fitting the projection 244 of the base fitting portion 249 to the main body 22, the plurality of stepped portions 241, 242 are brought into plane-to-plane contacts with the fitting base 222.

The projection 244 formed at the base portion of the rotational manipulator 24 (base fitting portion 249) is inserted through the fitting base 222. On the other hand, the stopper 224 is located around the projection 244. The stopper 224 serves to prevent the projection 244 inserted through the fitting base 222 from coming off from the fitting base 222.
The stopper 224 according to this embodiment has a U-shaped cross-section, and serves as a key member to be fitted in a recess 243 formed around the projection 244.
Accordingly, as shown in Fig. 5, when the projection 244 of the rotational manipulator 24 is inserted from the distal side through the opening 232 (see Fig. 6) of the main body 22, the stopper 244 is fitted in a radial direction to the recess 243 of the projection 244. The stopper 224 is larger in diameter than the opening 232, and hence inhibits the projection 244 from moving back and forth like a latch. Consequently, the base fitting portion 249 attached to the main body 22 is prevented from being removed to the distal side.

The stopper 224 biases the rotational manipulator 24 (base fitting portion 249) toward the main body 22, to the proximal side. Upon fitting the stopper 224 to the recess 243 of the base fitting portion 249, the stepped portions 241, 242 of the base fitting portion 249 are in contact with the fitting base 222 of the stepped shape with a predetermined axial force (normal force). Accordingly, the base fitting portion 249 and the main body 22 are connected so as to rotate relatively to each other, with a predetermined static friction force.

With the high-frequency treatment instrument 10 configured as above according to this embodiment, the orientation of the treatment element 60 can be prevented from accidentally fluctuating while moving the operating wire 30 with the controlling axial manipulator 26 or handling the manipulating unit 20.

When the operating wire 30 is made to rotate about the axis by rotating the rotational manipulator 24, only a part of the rotational angle achieved by the operating wire 30 through the rotational manipulator 24 is transmitted to the treatment element 60, because of a friction loss between the flexible tube 50 and the operating wire 30. In other words, when the treatment element 60 is oriented to a desired direction by operating the rotational manipulator 24, the operating wire 30 set immobile inside the flexible tube 50 is subjected to a predetermined torque.
When the axial manipulator 26 is made to slide toward the proximal side of the high-frequency treatment instrument 10 so as to pull the operating wire 30 in this state, the torsional rigidity of the operating wire 30 is improved in proportion to the tension applied thereto. Accordingly, when the operating wire 30 is pulled the operating wire 30 may reversely rotate in the flexible tube 50, and hence the treatment element 60 may shift from the desired orientation.
In the high-frequency treatment instrument 10 according to this embodiment, however, the rotational manipulator 24 and the main body 22 are in contact with each other via the plurality of stepped portions 241, 242, thus engaged with each other with a predetermined friction force. Accordingly, the rotational manipulator 24 and the main body 22 are kept from rotating relatively to each other by the static friction force when the axial manipulator 26 is made to slide so as to pull the operating wire 30, and therefore the operating wire 30 is prevented from reversely rotating. Consequently, the high-frequency treatment instrument 10 according to this embodiment can prevent the treatment element 60 from shifting from the desired orientation at the moment of performing a treatment such as resection of a diseased part by operating the axial manipulator 26.

### [Second embodiment]

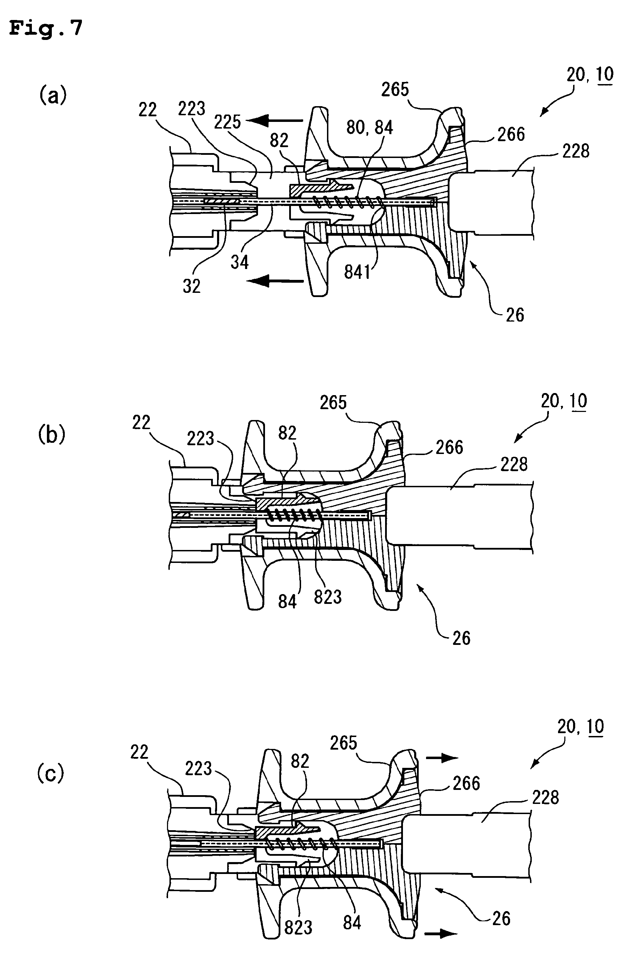

Figs. 7(a) to 7(c) are cross-sectional views of the manipulating unit 20 of the high-frequency treatment instrument 10 according to a second embodiment. Fig. 7(a) shows a state where the slider 265 is at a retreated position, Fig. 7(b) shows a state where the slider 265 is at the forwardmost position, and Fig. 7(c) shows a state where the operating wire 30 is slacked.

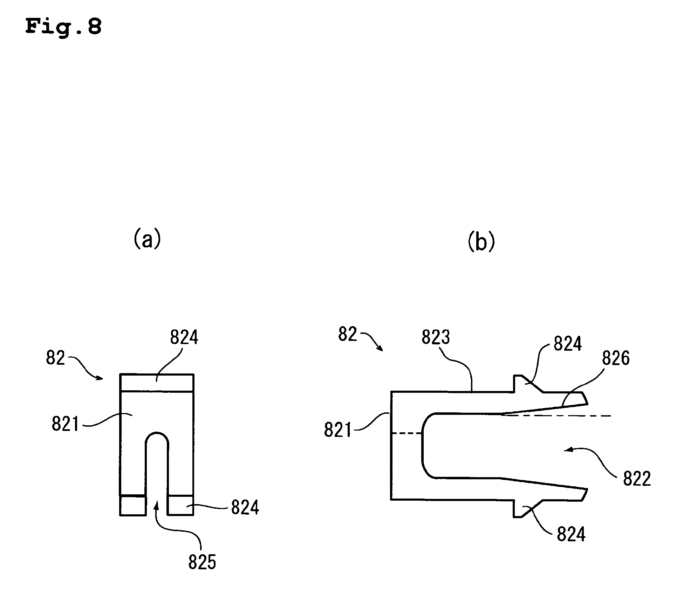

Figs. 8(a) and 8(b) constitute a two-view drawing of a holder 82 constituting a mitigator 80. Fig. 8(a) is a side view of the distal side of the holder 82, and Fig. 8(b) is a front view thereof.

The high-frequency treatment instrument 10 according to this embodiment further includes a mitigator 80 that applies a biasing force to the operating wire 30 that has axially moved, in an opposite axial direction.

In this embodiment, the expression "mitigator 80 applies a biasing force to the operating wire 30" is not limited to the case where the mitigator 80 and the operating wire 30 make direct contact with each other so as to transmit the biasing force. This embodiment also encompasses the case where the biasing force is indirectly transmitted from the mitigator 80 to the operating wire 30 through other components such as the slider fixer 266.

The high-frequency treatment instrument 10 according to this embodiment is distinctive in that the tension applied to the operating wire 30 through the operation of the axial manipulator 26 is alleviated by the mitigator 80. Such an arrangement contributes to improving the operability of the rotational manipulator 24 in the state where the operating wire 30 has been moved forward or backward. As stated above, when the slider 265 is moved so as to axially extend or compress the operating wire 30, the torsional rigidity of the operating wire 30 increases the greater the tension applied thereto is. Therefore, biasing the operating wire 30 in a direction opposite to the moving direction of the axial manipulator 26 mitigates the tension, so that the natural torsional rigidity of the operating wire 30 can be restored. Such an arrangement prevents the torque necessary for operating the rotational manipulator 24 from excessively increasing.

Here, the tension of the operating wire 30 refers to both of the compressing force and the tensile force applied to the operating wire 30, in this embodiment.

As stated with reference to the first embodiment, the high-frequency treatment instrument 10 allows the treatment element 60 to be manipulated free from an impact of the self weight of the high-frequency cable 70, because of the configuration in which the axial manipulator 26 and the rotational manipulator 24 are provided independently from the main body 22 (see Fig. 1). Employing further the mitigator 80 to thereby alleviate the tension of the operating wire 30 upon moving the same forward or backward according to this embodiment allows the rotational torque necessary for rotating the treatment element 60 to be reduced, thus further improving the operability of the rotational manipulator 24.

Such an advantage can be prominently appreciated, in particular, in the case where the treatment element 60 is opened and its angle is adjusted to the diseased part, which is a typical procedure with a scissors type instrument or a snare instrument. In this embodiment, the treatment element 60 is exemplified by the scissors type element (see Fig. 3).

The treatment element 60 according to this embodiment includes a plurality of scissor pieces (first scissor piece 61 and second scissor piece 62) configured to open and close with respect to each other by moving the operating wire 30 back and forth with the axial manipulator 26.
The mitigator 80 applies a biasing force to the operating wire 30 in a backward or forward direction, when the operating wire 30 is moved forward or backward so that the first scissor piece 61 and the second scissor piece 62 are open from each other.

More specifically, the operating wire 30 is pulled toward the proximal side when the slider 265 is at the retreated position as shown in Fig. 7(a), the first scissor piece 61 and the second scissor piece 62 are closed (see Fig. 3(b)).
When the slider 265 is moved forward, the operating wire 30 is pushed toward the distal side. With such a movement, the first scissor piece 61 and the second scissor piece 62 pivot about the first pin 65, so as to present an open state (see Fig. 3(a)).

Here, the scissors type treatment element 60 may be a parallel slide type in which the first scissor piece 61 and the second scissor piece 62 move toward and away from each other in a radial direction of the flexible tube 50 maintaining a parallel orientation, instead of the pivotal type as shown in Figs. 3(a) and 3(b).

Fig. 3(a) and Fig. 7(b) illustrate a state where the operating wire 30 has been moved all the way so that the first scissor piece 61 and the second scissor piece 62 are open from each other to the maximum angle.
Then as shown in Fig. 7(c), the operating wire 30 is moved backward or forward (in this embodiment, backward) by the biasing force of the mitigator 80 after the maximum open state of the treatment element 60, so that the first scissor piece 61 and the second scissor piece 62 present an intermediate open state (not shown) in which the aperture (opening angle) is decreased from the maximum open state. Here, the intermediate open state of the treatment element 60 refers to any state other than the maximum open state and a completely closed state.

Referring to Figs. 7(a) to 7(c) and Fig. 8, the high-frequency treatment instrument 10 according to this embodiment will be described in further details.

The mitigator 80 is composed of a combination of a holder 82 having a channel-shaped vertical cross-section with the opening oriented to a lateral side, and a biasing spring 84. The mitigator 80 may be composed of a plurality of parts as in this embodiment, or may be formed of a single-piece material.

The biasing spring 84 is a helical spring. The outer diameter of the biasing spring 84 is smaller than the opening width of the slit 225 of the main body 22. The biasing spring 84 is attached around the lead pipe 34, and elastically extends and contracts in the extending direction of the lead pipe 34, i.e., the axial direction of the manipulating unit 20 (left and right in Figs. 7(a) to 7(c)).
The base end portion 841 of the biasing spring 84 may be fixed to the lead pipe 34 or the slider fixer 266, or unconnected to each other.

The holder 82 serves to axially compress the biasing spring 84 to thereby bias the axial manipulator 26 so as to move away from the main body 22, when the axial manipulator 26 and the main body 22 come axially closer to each other than a predetermined distance.
The holder 82 includes a flat pressing surface 821 on the front side thereof oriented toward the distal side of the manipulating unit 20 (left side in Fig. 8(b)), and an opening 822 on the rear side (right side in Fig. 8(b)). A pair of elastic pieces 823 formed generally parallel to each other across the opening 822 each include an latch nail 824 formed so as to outwardly protrude in a vertical direction. The slope angle of the latch nail 824 on the side of the opening (rear side) is milder than the slope angle thereof on the front side.
A tapered portion 826 is formed on the inner surface of the rear end portion of the elastic piece 823, such that the diameter of the opening 822 increases toward the proximal side.

The holder 82 includes a groove 825 having an opening oriented downward. The width of the groove 825 is wider than the outer diameter of the lead pipe 34, and smaller than the outer diameter of the biasing spring 84.

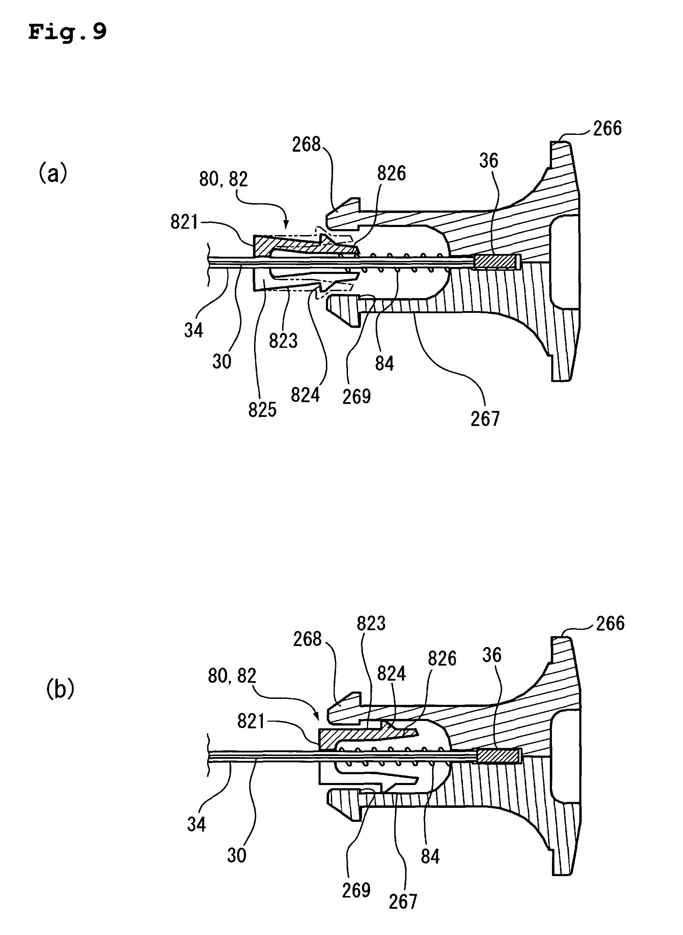

Fig. 9(a) is a vertical cross-sectional view showing a state where the mitigator 80 (holder 82 and biasing spring 84) is about to be fitted, and Fig. 9(b) is a vertical cross-sectional view showing a state where the mitigator 80 has been fitted. In Fig. 9(a), dash-dot-dot lines show the holder 82 in the natural state, and solid lines show the holder 82 with the latch nail 824 insert-fitted.

Figs. 9(a) and 9(b) illustrate the slider fixer 266, the operating wire fixing base 36 buried in the slider fixer 266, the lead pipe 34 extending toward the distal side from the operating wire fixing base 36, and the operating wire 30 inserted through the lead pipe 34. The slider 265 and the main body 22 are not shown.

First, the biasing spring 84 is attached from the distal side (left in Fig. 9(a)) of the lead pipe 34. The biasing spring 84 is attached so as to slide with respect to the lead pipe 34.
Then the lead pipe 34 is inserted in the groove 825 with the pressing surface 821 of the holder 82 oriented toward the distal side. At this stage, the holder 82 is located so as to straddle on the lead pipe 34 as shown in Fig. 9(a).

Thereafter, the elastic pieces 823 of the holder 82 are pressed in a vertical direction such that the latch nails 824 sink with respect to the pressing surface 821, and the holder 82 is inserted between a pair of arms 267 of the slider fixer 266. At this moment, since the opening side (rear side) of the latch nail 824 is gently inclined, the latch nails 824 are biased toward the axial center by the front end portion 268 of the slider fixer 266 simply by pressing the holder 82 to the rear side, and the elastic pieces 823 are inwardly deflected. Thus, the latch nails 824 become able to pass between the pair of front end portions 268.

Here, since the elastic piece 823 includes the tapered portion 826 formed on the inner surface of the rear end portion, the tapered portion 826 becomes parallel to the lead pipe 34 when the elastic piece 823 is deflected, as shown in Fig. 9(a). At this moment, the distance between the tapered portions 826 is larger than the outer diameter of the biasing spring 84, and therefore the holder 82 can intrude through between the arms 267 along the outer periphery of the biasing spring 84.

As shown in Figs. 9(a) and 9(b), a stepped portion 269 is provided between the front end portion 268 and the arm 267 of the slider fixer 266. When the latch nail 824 passes the stepped portion 269, the latch nails 824 again protrude with respect to the pressing surface 821 because of the elastic restoring force of the elastic piece 823, as shown in Fig. 9(b). Thus, the latch nail 824 and the stepped portion 269 are engaged with each other, so that holder 82 is restricted from coming off from the slider fixer 266.
Through the foregoing procedure, the holder 82 and the biasing spring 84 can be fitted inside the slider fixer 266.

Referring again to Figs. 7(a) to 7(c), the working of the mitigator 80 according to this embodiment will be described in details.

The holder 82 and the biasing spring 84 are accommodated in the slit 225 of the manipulating unit 20, and fitted inside the slider fixer 266.
On the other hand, the main body 22 includes a backward projection 223 formed so as to axially protrude from the distal side of the axial manipulator 26 toward the inside of the slit 225. The backward projection 223 has a tapered shape such that the width thereof decreases toward the proximal side. The proximal side end face of the backward projection 223 is flat and parallel to the pressing surface 821 of the holder 82.

As shown in Fig. 7(a), the holder 82 and the biasing spring 84 are located between the main body 22 and the axial manipulator 26. In other words, the mitigator 80 (holder 82 and biasing spring 84) is resiliently interposed between the main body 22 and the axial manipulator 26.

When the slider 265 is at the retreated position as shown in Fig. 7(a), the biasing spring 84 assumes its natural length and hence the main body 22 and the axial manipulator 26 are not subjected to a biasing force. Also, the first scissor piece 61 and the second scissor piece 62 of the treatment element 60 are closed, as stated above (see Fig. 3 (b)). The treatment element 60 and the flexible tube 50 are inserted through a forceps channel of an endoscope (not shown) in this state.

Here, an endoscope normally includes a plurality of forceps channels. In the forceps channels, optical systems such as a fiber scope and a charge-coupled device (CCD) camera are inserted, in addition to the high-frequency treatment instrument 10 according to this embodiment. In addition, a cylindrical hood may be attached to the distal end portion of the endoscope. Attaching the hood to the distal end portion of the endoscope so as to protrude therefrom allows the relative position between a diseased part and the optical system to be fixed while maintaining unchanged the distance therebetween.

When the treatment element 60 sticks out from the distal end portion of the forceps channel of the endoscope, the position and rotational angle of the treatment element 60 inside the hood can be monitored through the optical system. Then the rotational manipulator 24 is rotated about the axis so as to adjust the rotational angle of the treatment element 60 to the diseased part, monitoring the treatment element 60 through the optical system. A specific example of the manipulation of the treatment element 60 will be described here below.

First, the operator passes a first finger (thumb) through the auxiliary ring 262 (see Fig. 1), and holds the slider 265 with other fingers to move the axial manipulator 26 forward all the way through its stroke range, as shown in Fig. 7(b). Such a state will be herein referred to as forwardmost position of the axial manipulator 26.
In the forwardmost position of the axial manipulator 26, the operating wire 30 is moved forward with the axial manipulator 26, so that the first scissor piece 61 and the second scissor piece 62 present the maximum open state (see Fig. 3(a)). Accordingly, the V-shape formed by the first scissor piece 61 and the second scissor piece 62 can be monitored through the optical system.

In the case where the treatment element 60 is of a scissors type, a widthwise size (vertical direction in Fig. 3(b)) and a thicknesswise size (depthwise direction in Fig. 3 (b)) are close when the scissors are closed, and therefore it is generally difficult to visually recognize the rotational angle of the treatment element 60 through the optical system. Accordingly, it is preferable to monitor the rotational angle through the optical system with the treatment element 60 opened, as in this embodiment.

At this stage, the holder 82 is pressed to the proximal side (to the right in Fig. 7(b)) by the backward projection 223, and the elastic pieces 823 are made to abut against the slider fixer 266. Since the natural length of the biasing spring 84 is longer than the elastic piece 823, the biasing spring 84 is elastically compressed when the holder 82 is pressed deeper into the slider fixer 266. Accordingly, the biasing spring 84 exerts an axially backward biasing force on the operating wire 30 through the slider fixer 266 and the lead pipe 34. However, as long as the fingers of the operator hold the axial manipulator 26, the axial manipulator 26 stays immobile at the forwardmost position, and the biasing spring 84 remains compressed.

When the operator releases the axial manipulator 26 from the fingers, the biasing spring 84 causes the holder 82 and the slider fixer 266 to slide so as to move away from each other, with an elastic restoring force. Thus the slider fixer 266 is moved backward as shown in Fig. 7(c).

Here, the elastic restoring force gained by the biasing spring 84 when the axial manipulator 26 is at the forwardmost position is greater than the total of a maximum static friction force between the operating wire 30 and lead pipe 34 and slider fixer 266, and the flexible tube 50 and main body 22. Therefore, when the operator simply releases the axial manipulator 26 from the fingers, the axial manipulator 26 slightly moves backward.
Then the tensile force (compressive force) of the operating wire 30 pressed to the forwardmost position is at least partially offset, so that the operating wire 30 becomes substantially slacked.
In this embodiment, the state where the axial manipulator 26 is at an intermediate position other than the forwardmost and the backwardmost position of the stroke range will be referred to as slacked state of the operating wire 30.

In the slacked state of the operating wire 30 shown in Fig. 7(c), since the first scissor piece 61 and the second scissor piece 62 (see Fig. 3(a)) are open from each other, the orientation of the treatment element 60 can be monitored through the optical system. In addition, the aperture between the first scissor piece 61 and the second scissor piece 62 in the slacked state of the operating wire 30 is smaller than the aperture in the maximum open state. Therefore, the tip portions of the first scissor piece 61 and the second scissor piece 62 can be prevented from interfering with the inner surface of the hood.

As described above, with the high-frequency treatment instrument 10 according to this embodiment, although the operator moves the axial manipulator 26 all the way to the forwardmost position, the aperture of the treatment element 60 automatically decreases so that the operating wire 30 is slacked, when the operator only releases the axial manipulator 26 from the fingers. Accordingly, when the operator proceeds to the operation of the rotational manipulator 24 the tension of the operating wire 30 is mitigated, and therefore the operator can smoothly operate the rotational manipulator 24 without the need to apply a large rotational torque. Further, since the treatment element 60 is in the intermediate open state when the rotational manipulator 24 is about to be operated, the treatment element 60 can be prevented from physically interfering with the hood without compromise in visibility through the optical system.

In particular, in the case where the axial manipulator 26 has a large stroke range such that the axial manipulator 26 can still move forward even after the treatment element 60 reaches the maximum open state, a large amount of compressive force may be imposed to the operating wire 30 upon moving the axial manipulator 26 further forward. However, by utilizing the mitigator 80 so as to mitigate or eliminate the compressive force against the operating wire 30 as in this embodiment, the rotational torque necessary for operating the rotational manipulator 24 can be significantly minimized.

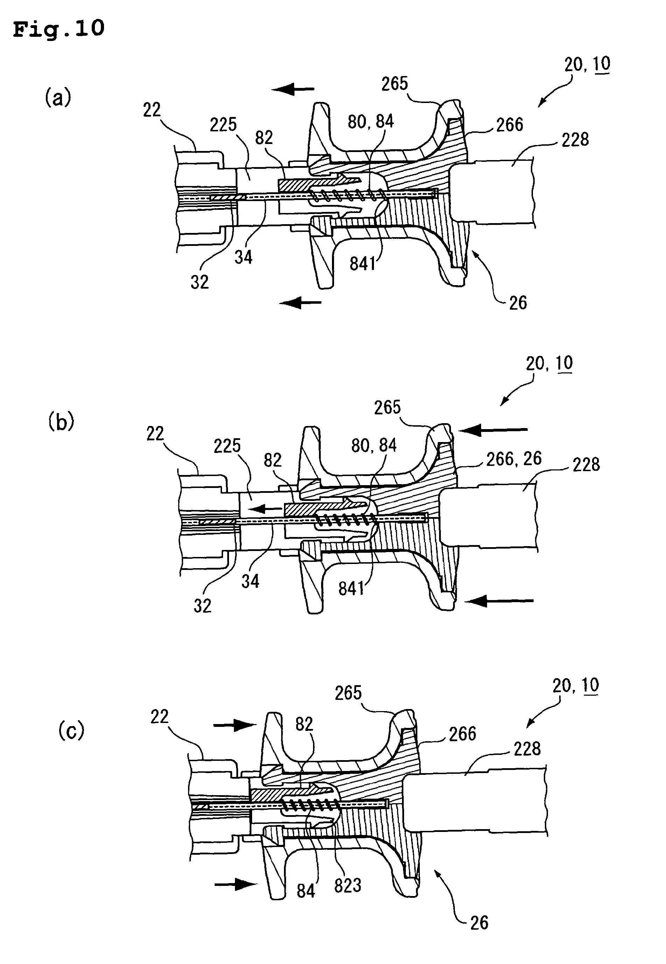

Figs. 10(a) to 10(c) are cross-sectional views showing a variation of the high-frequency treatment instrument 10 according to the second embodiment. In the high-frequency treatment instrument 10 according to this variation, the mitigator 80 includes the holder 82 engaged with the operating wire 30 with a predetermined engaging force so as to restrict the back-and-forth movement of the axial manipulator 26, and a biasing member (biasing spring 84) resiliently interposed between the holder 82 and the axial manipulator 26. In the high-frequency treatment instrument 10 according to this variation, the holder 82 and the operating wire 30 are disengaged from each other thus allowing the axial manipulator 26 to axially move, in the case where a load greater than a predetermined engaging force is axially imposed on the axial manipulator 26.

The holder 82 is directly or indirectly engaged with the operating wire 30. In this variation, the holder 82 is fitted to the lead pipe 34 through which the operating wire 30 is inserted, and the holder 82 frictionally slides with respect to the lead pipe 34. Fig. 10(a) shows a state where the axial manipulator 26 is restricted from moving forward because of the engagement between the holder 82 and the lead pipe 34 (operating wire 30). As shown therein, the holder 82 is engaged with the lead pipe 34 through a static friction force and the biasing spring 84 is compressed, when the axial manipulator 26 starts to move from the retreated position toward the distal side (to the left in Fig. 10(a)).

The engagement between the holder 82 and the operating wire 30 may be achieved by friction as described above, or by a projection and recess that can be engaged or disengaged by a predetermined axial external force.

As the axial manipulator 26 moves further forward, the compression amount of the biasing spring 84 increases. When the elastic restoring force of the biasing spring 84 is balanced with the maximum static friction force between the holder 82 and the lead pipe 34, the holder 82 frictionally slides with respect to the lead pipe 34 toward the distal side, as shown in Fig. 10(b). Fig. 10(b) illustrates how the axial manipulator 26 is moving forward.

The pressing surface 821 of the holder 82 according to this variation protrudes to the distal side with respect to the front end portion 268 of the slider fixer 266, when the holder 82 is pressed to the deepest position between the arms 267 of the slider fixer 266 (see Figs. 9(a) and 9(b)).

When the axial manipulator 26 moves further forward from the state shown in Fig. 10 (b) and the pressing surface 821 of the holder 82 is brought into contact with the main body 22, the biasing spring 84 exerts an elastic force toward the proximal side on the slider fixer 266 (see Fig. 10(c)).

Accordingly, the axial manipulator 26 is moved slightly backward from the forwardmost position, so that a part of the pressing force against the operating wire 30 is removed and the operating wire 30 becomes slacked. Fig. 10(c) therefore illustrates the slacked state of the operating wire 30. At this moment, the first scissor piece 61 and the second scissor piece 62 (see Fig. 3(a)) slightly reduces the aperture from the maximum open state. Therefore, the rotational torque necessary for operating the rotational manipulator 24 is reduced, and physical interference between the treatment element 60 (see Fig. 1) and the hood can be avoided.

The high-frequency treatment instrument 10 offers a unique and prominent advantage when used for a body cavity that is curved or bent (hereinafter simply defined as curved without strict distinction therebetween).
In the case where the body cavity (or lumen of a catheter inserted therethrough) is curved, when the flexible tube 50 and the operating wire 30 of the high-frequency treatment instrument 10 (hereinafter, see Fig. 1) are inserted the operating wire 30 advances relatively to the flexible tube 50. This is because the operating wire 30 inserted through the flexible tube 50 moves over a longer path length than the inner side of the curved portion of the flexible tube 50. Accordingly, as the flexible tube 50 and the operating wire 30 are further curved, the operating wire 30 advances with respect to the flexible tube 50 while the treatment element 60 at the distal end portion of the flexible tube 50 remains closed, and the axial manipulator 26 is drawn toward the distal side.

In contrast, with the high-frequency treatment instrument 10 according to this variation, although the axial manipulator 26 is slightly drawn toward the distal side so that the biasing spring 84 is compressed, the holder 82 frictionally engaged with the lead pipe 34 serves as a stopper so as to restrict the axial manipulator 26 from moving forward, as shown in Fig. 10(a). Therefore, with the high-frequency treatment instrument 10 according to this variation, the axial manipulator 26 is kept from moving all the way to the tip position of the slit 225, even though the flexible tube 50 and the operating wire 30 are inserted in a curved body cavity or lumen. In other words, the maximum static friction force constituting the engaging force between the holder 82 and the lead pipe 34 is greater than the elastic restoring force of the biasing spring 84 generated at the advanced position of the axial manipulator 26 in the case where the flexible tube 50 and the operating wire 30 are curved.
Thus, even in the case where the high-frequency treatment instrument 10 is inserted in a curved body cavity or lumen and the treatment element 60 has reached the vicinity of the diseased part, a surplus for further forward stroke is still left available for the axial manipulator 26 so as to open the treatment element 60.

However, the external force exerted by the operator to move the axial manipulator 26 forward is by far greater than the engaging force between the holder 82 and the lead pipe 34. Accordingly, as shown in Fig. 10(b), upon moving the axial manipulator 26 forward, the holder 82 moves forward with the slider fixer 266 with respect to the lead pipe 34. Therefore the operating wire 30 advances relatively to the main flexible tube 50 fixed to the body 22, so that the treatment element 60 is opened.

In the case where the axial manipulator 26 has reached the forwardmost position, the axial manipulator 26 is moved slightly backward by the resilient force of the biasing spring 84 as shown in Fig. 10(c), and the operating wire 30 becomes slacked as stated above.

Thus, with the high-frequency treatment instrument 10 according to this variation, even though the flexible tube 50 is inserted in a curved body cavity or lumen, the mitigator 80 properly restricts the forward movement of the axial manipulator 26, so that the axial manipulator 26 and the mitigator 80 can fully perform the respective functions.

This application claims priority based on Japanese Patent Application No. 2009-123077 filed on May 21, 2009, the content of which is incorporated hereinto in its entirety.

## Claims

1. A high-frequency treatment instrument comprising:
a flexible tube to be inserted in a body cavity;
an electrically conductive operating wire inserted through the flexible tube so as to move back and forth;
a treatment element provided at a distal end portion of the operating wire and configured to treat a diseased part by application of a high-frequency current; and
a manipulating unit attached to a proximal end portion of the flexible tube and configured to move the operating wire back and forth to thereby manipulate the treatment element, and to rotate the operating wire torsionally about a longitudinal axis thereof to thereby rotate the treatment element,
wherein the manipulating unit includes a main body being attached a high-frequency cable that supplies a high-frequency current to the treatment element, a rotational manipulator rotatably attached to a distal side of the main body so as to rotate the operating wire about the longitudinal axis thereof, and an axial manipulator attached to a proximal side of the main body so as to axially move to thereby cause the operating wire to move back and forth.

2. The high-frequency treatment instrument according to Claim 1,
wherein the main body includes a conductive pipe made of a metal buried therein, the conductive pipe being electrically connected to the high-frequency cable and through which the operating wire is inserted, and
the operating wire includes a cylindrical conductor that slides on the conductive pipe maintaining electrical connection to the conductive pipe.

3. The high-frequency treatment instrument according to Claim 2, further comprising a lead pipe made of a metal attached to an outer periphery of the proximal end portion of the operating wire, and the conductor is provided on an outer surface of the lead pipe.

4. The high-frequency treatment instrument according to any one of Claims 1 to 3, further comprising a mitigator that applies a biasing force to the operating wire in a direction opposite to an axial movement thereof.

5. The high-frequency treatment instrument according to Claim 4,
wherein the treatment element includes a plurality of scissor pieces caused to open and close with respect to each other by a movement of the axial manipulator to move the operating wire back and forth, and
the mitigator applies the biasing force to the operating wire when the scissor pieces are made to open by a forward or backward movement of the operating wire.

6. The high-frequency treatment instrument according to Claim 5,
wherein the operating wire is caused to move backward or forward by the biasing force of the mitigator, from a maximum open state realized by axial movement of the operating wire where an aperture of the scissor pieces is largest, and the scissor pieces present an intermediate open state where the aperture is smaller than that of the maximum open state.

7. The high-frequency treatment instrument according to any one of Claims 4 to 6,
wherein the mitigator is resiliently interposed between the main body and the axial manipulator.

8. The high-frequency treatment instrument according to Claim 7,
wherein the mitigator includes a holder engaged with the operating wire with a predetermined engaging force to thereby restrict a back-and-forth movement of the axial manipulator, and a biasing member resiliently interposed between the holder and the axial manipulator, and
the holder becomes disengaged from the operating wire when a load greater than the predetermined engaging force is applied to the axial manipulator in the axial direction, to thereby allow the back-and-forth movement of the axial manipulator.

9. The high-frequency treatment instrument according to any one of Claims 1 to 8,
wherein the rotational manipulator includes a plurality of stepped portions formed on a proximal end portion thereof,
the main body includes a multiple-stage fitting base formed on a distal end portion thereof, the fitting base being attached the rotational manipulator, and
the plurality of stepped portions make plane-to-plane contacts with the fitting base.

10. The high-frequency treatment instrument according to Claim 9,
wherein the rotational manipulator includes a projection formed on the proximal end portion thereof, the projection being inserted through the fitting base, and
the projection includes a stopper formed therearound so as to prevent the projection from coming off from the fitting base.

11. The high-frequency treatment instrument according to Claim 10,
wherein the stopper biases the rotational manipulator toward the proximal side of the main body.

12. The high-frequency treatment instrument according to any one of Claims 1 to 11,
wherein the axial manipulator has an axially symmetrical shape.

13. The high-frequency treatment instrument according to Claim 12,
wherein the high-frequency treatment instrument further includes an annular auxiliary ring pivotally attached to the main body and having an opening oriented in a direction intersecting with the axial direction, and
the axial manipulator is movable in the axial direction being restricted from rotating about the axis with respect to the main body.

14. The high-frequency treatment instrument according to any one of Claims 1 to 13,
wherein the rotational manipulator is circumferentially exposed on an outer surface of the main body.
